# EUROPÄISCHE PATENTANMELDUNG

(11) **EP 1 440 685 A1**
(43) Veröffentlichungstag der Anmeldung: **28.07.2004**
(21) Anmeldenummer: 04001129.8
(22) Anmeldetag: 20.01.2004
(51) Int. Cl.: A61K 7/48

(54) **Kosmetische oder dermatologische Zubereitungen mit Perlglanzoptik**

(30) Priorität: 20.01.2003 DE 10301836
(71) Anmelder: Beiersdorf AG, 20253 Hamburg (DE)
(72) Erfinder: Kohlhase, Silke (ehemals Füller), 22523 Hamburg (DE); Bleckmann, Andreas, 22926 Ahrensburg (DE); Riedel, Heidi, 22529 Hamburg (DE); von Thaden, Stefanie, 20255 Hamburg (DE)
(74) Vertreter: Hartmann, Jost, Dr.

(57) **Zusammenfassung**

Die kosmetische oder dermatologische Zubereitung umfasst
(I) bis zu 12 Gew.%, bezogen auf das Gesamtgewicht der Zubereitungen, einer oder mehrerer C₁₂-C₄₀ Fettsäuren,
(II) 0 bis maximal 1 Gew.%, bezogen auf das Gesamtgewicht der Zubereitungen, einer oder mehrerer C₁₂-C₄₀ Fettalkohole
(III) 0,01 bis 10 Gew.%, bezogen auf das Gesamtgewicht der Zubereitungen, amphiphile Polymere und/oder assoziative Polymere und/oder Siloxane Elastomere und
(IV) Natronlauge
   oder

(I) maximal 10 Gew.% .%, bezogen auf das Gesamtgewicht der Zubereitungen, einer oder mehrerer C₁₂-C₄₀ Fettsäuren,
(II) 0,1 bis 10 Gew.% .%, bezogen auf das Gesamtgewicht der Zubereitungen, einer oder mehrerer C₁₂-C₄₀ Fettalkohole,
(III) 0,01 bis 10 Gew.%, bezogen auf das Gesamtgewicht der Zubereitungen, amphiphile Polymere und/oder assoziative Polymere und/oder Siloxane Elastomere,
(IV) Natronlauge sowie
(V) Bis zu 10 Gew.%, bezogen auf das Gesamtgewicht der Zubereitungen, C₁₂-C₄₀ polyethoxylierte (POE) Fettsäureester mit einer POE-Kettenlänge von 10-100 und
(VI) gegebenenfalls niedermolekulare Tenside

Die Zubereitung weist einen optisch ansprechenden Perlglanz auf, ist lagerstabil und äußerst hautverträglich.

## Beschreibung

Die Erfindung betrifft kosmetische Zubereitungen mit einem Perlglanzeffekt und einer guten Hautverträglichkeit sowie deren Verwendung.

Übliche kosmetische Darreichungsformen und Zubereitungen sind häufig Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuss eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerken zurückgeführt. Um die Metastabilität von Emulsionen gewährleisten zu können, sind in der Regel grenzflächenaktive Substanzen, also Emulgatoren, nötig.

Emulsionen aus dem Stand der Technik weisen einen Perlglanzeffekt auf, wie es beispielsweise in WO 0110403, WO 9010429, DE 19921186 oder DE 19944545 beschrieben ist. Diese kosmetischen Zubereitungen enthalten Mono- und Difettsäureester des Glycerins oder Glycols, wie beispielsweise Glycerylstearate, - laurate oder myristate, um einen Perlglanz der Zubereitung zu gewährleisten. Diese Fettsäureester bilden in O/W - Emulsionen geordnete, lamellare Strukturen mit lyotropen, flüssigkristallinen Eigenschaften. Dies führt zu einer als Perlglanz bezeichneten optischen Eigenschaft der sie enthaltenden Emulsionen.

Zubereitungen mit Perlglanzoptik ohne Zusatz dieser Fettsäureester sind nicht oder nur schwer zugänglich.

Kosmetische Zubereitungen und Emulsionen mit Perlglanzoptik basierend auf dem Emulgator "Stearinsäure/ Palmitinsäure" sind seit langem bekannt. Nicht möglich war bisher die Formulierung von Perlglanz-Emulsionen, die das Neutralisationsmittel NaOH und Anteile an Fettsäuren unterhalb 12 Gew.% aufweisen.

Aufgrund dessen zeigen die aus dem Stand der Technik bekannten und im Markt befindlichen Perlglanz-Emulsionen eine sehr schlechte Hautverträglichkeit.

Ein optischer Perlglanzeffekt wird in kosmetischen Zubereitungen nach dem Stand der Technik ausschließlich durch Neutralisation mit Triethanolamin oder Kalilauge erzielt. Mit Natronlauge waren bisher keine Systeme mit perlmuttartiger Optik herstellbar, wie beispielsweise in Modern Cosmeticology, Volume one, 1996, Ralph G. Harry, F.R.I.C, 1962, S. 115-119 angeführt wird. Darin wird ebenfalls deutlich, dass Lipide und Wachse den Perlglanz in Emulsionen inhibiieren. Es werden Perlglanz-Emulsionen beschrieben, die sehr geringe, bis zu maximal 3 Gew.%, Anteile an Lipiden und oder lipohilen Konsistenzgeber enthalten.
Zudem ist bekannt, dass der alleinige Einsatz von Natronlauge in diesen Systemen keine ausreichende Lagerstabilität der kosmetischen Zubereitung gewährleistet, wie beispielsweise schon in The American Perfumer, April 1945, "Manufacturing Vanishing Cream", J.S. Shukla dargelegt wird. Darin wird auch offenbart, dass Perlglanzemulsionen ausschließlich mittels hoher Einsatzkonzentration an Fettsäuren erzielt werden können. So werden beispielsweise 16-25 Gew.% an Fettsäuren, wobei 13,3 Gew.% verseift sein sollten, verwendet. Bekannt ist jedoch auch, beispielsweise aus Kosmetologie, 3. Auflage, 1976, Dr. J.Jellinek (S. 235-239), dass gerade dieser hohe Seifengehalt von 13,3 Gew.% zu schlechten Hautverträglichkeiten führt.

Aufgabe der vorliegenden Erfindung ist es eine kosmetische Zubereitung bereit zu stellen, die einen optisch ansprechenden Effekt, insbesondere einen Perlglanzeffekt aufweist, eine ausreichende Lagerstabilität und vor allem eine gute Hautverträglichkeit aufweist. Darüber hinaus ist es die Aufgabe der vorliegenden Erfindung eine kosmetische Zubereitung bereit zu stellen, die den Stand der Technik bereichert.

Gelöst wird das Bündel an Aufgaben durch eine kosmetische Zubereitung nach Anspruch 1 oder 2. Die Erfindung umfasst weiterhin die Verwendung der erfindungsgemäßen Zubereitungen. In den Unteransprüchen sind bevorzugte Ausführungsformen der erfindungsgemäßen Zubereitungen dargelegt.

Es wurde überraschenderweise gefunden, dass kosmetische oder dermatologische Zubereitung umfassend
(I) bis zu 12 Gew.%, bezogen auf das Gesamtgewicht der Zubereitungen, einer oder mehrerer C₁₂-C₄₀ Fettsäuren,
(II) 0 bis maximal 3 Gew.%, bezogen auf das Gesamtgewicht der Zubereitungen, einer oder mehrerer C₁₂-C₄₀ Fettalkohole
(III) 0,01 bis 10 Gew.%, bezogen auf das Gesamtgewicht der Zubereitungen, amphiphile Polymere und/oder assoziative Polymere und/oder Siloxan-Elastomere und
(IV) Natronlauge
oder Zubereitungen umfassend
(I) maximal 10 Gew.% .%, bezogen auf das Gesamtgewicht der Zubereitungen, einer oder mehrerer C₁₂-C₄₀ Fettsäuren,
(II) 0,1 bis 10 Gew.% .%, bezogen auf das Gesamtgewicht der Zubereitungen, einer oder mehrerer C₁₂-C₄₀ Fettalkohole,
(III) 0,01 bis 10 Gew.%, bezogen auf das Gesamtgewicht der Zubereitungen, amphiphile Polymere und/oder assoziative Polymere und/oder Siloxane Elastomere,
(IV) Natronlauge sowie
(V) zusätzlich C₁₂-C₄₀ polyethoxylierte (POE) Fettsäureester mit einer POE-Kettenlänge von 10-100.
die Lösung all der gestellten Aufgaben ermöglicht.
Kennzeichnend ist, dass derartige perlglänzende Zubereitungen im Sinne der vorliegenden Erfindung vorteilhaft frei sind von Mono- und/oder Difettsäureester des Glycerin und/oder Glycols. Diese üblicherweise genutzten Emulgatoren bzw. Perlglanzgeber werden vorteilhafterweise nicht eingesetzt, um den erfindungsgemäßen Perlglanzeffekt in obigen Formulierungen zu gewährleisten. Insbesondere bevorzugt sind erfindungsgemäße Zubereitungen, welche kein Glycerylstearat, Glyceryldistearat, Glycerylisostearat, Glyceryldiisostearat, Glyceryloleat, Glycerylpalmitat, Glycerylmyristat, Glyceryllanolat und/oder Glyceryllaurat enthalten.

Der wesentliche Vorteil der erfindungsgemäßen Zubereitungen liegt in der erstmalig aufgezeigten Möglichkeit, dass Natronlauge als ausschließliches Neutralisationsmittel enthalten sein kann ohne Einbussen hinsichtlich Langzeitstabilität, der kosmetischen Perlglanzoptik und vor allem der Hautverträglichkeit hinzunehmen.

Als C₁₂-C₄₀ Fettsäuren (I) kommen ganz-, teil- oder nicht neutralisierte, verzweigte und/oder unverzweigte, gesättigte und/oder ungesättigte Fettsäuren mit einer Kettenlänge von 12 bis 40 Kohlenstoffatomen in Frage.
Die Fettsäure(n) werden vorzugsweise gewählt aus der Gruppe der Säuren, welche ganz oder teilweise mit üblichen Alkalien (wie z. B. Natrium- und/oder Kaliumhydroxid, Natrium- und/oder Kaliumcarbonat) neutralisiert sind. Besonders vorteilhaft sind beispielsweise Stearinsäure und Stearate, Isostearinsäure und Isostearate, Palmitinsäure und Palmitate sowie Myristinsäure und Myristate. Durch die erfindungsgemäßen Zubereitungen ist es erstmals möglich auf den Einsatz von Mono- und/oder Triethanolamin als Neutralisationsmittel zu verzichten. Hierdurch wird eine verbesserte Hautverträglichkeit und dennoch gute Langzeitstabilität und hervorragender Perlglanz erzielt.

Bevorzugt werden C16/C18 Fettsäuren und Mischungen, insbesondere im eutektischen Gemisch, ganz besonders bevorzugt Stearin- und Palmitinsäure, eingesetzt.
Bei den eingesetzten Fettsäuren beträgt der verseifte Anteil bevorzugt max. 9%.

Die C₁₂-C₄₀ Fettsäuren (I) werden zu einem Anteil von bis zu 12 Gew.%, bevorzugt von 0,1-10 Gew.%, bezogen auf die Gesamtzubereitung eingesetzt.

Als C₁₂-C₄₀ Fettalkohole (II) werden Alkohole gewählt aus der Gruppe der gesättigten und/oder ungesättigten, verzweigten und/oder unverzweigten Fettalkohole mit einer Kettenlänge von 12 bis 40 Kohlenstoffatomen. Bevorzugt werden C14-C20-Fettalkohole ausgewählt.
Die Fettalkohole werden erfindungsgemäß vorzugsweise aus der folgenden Gruppe gewählt: Myristyl Alkohol Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Die C₁₂-C₄₀ Fettalkohole (II) werden zu einem Anteil von bis zu 10 Gew.%, bevorzugt von 0,1 - 5 Gew.%, bzw. bis zu 3 Gew.%, bezogen auf die Gesamtzubereitung, eingesetzt.

Als C₁₂-C₄₀ POE-Fettsäureester (V) werden polyethoxylierte Fettsäurester mit einer Kettenlänge von 12 bis 40 Kohlenstoffatomen und mit einem Ethoxylierungsgrad von 10 bis 100 gewählt. Aus der Gruppe der polyethoxylierten Fettsäurester werden vorzugsweise gewählt: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Insbesondere bevorzugt sind PEG-40 Stearate.

Die C₁₂-C₄₀ POE-Fettsäureester (V) werden zu einem Anteil von bis zu 10 Gew.%, bevorzugt von 0,1 - 5 Gew.%, bezogen auf die Gesamtzubereitung eingesetzt.

In einer bewährten Zubereitung liegen die Bestandteile I, II und V im Verhältnis (I : II : V) 5:1:1 bis 1:1:5 vor. Besonders bevorzugt sind Verhältnisse I : II : V im Bereich 3:1:1 bis 3:1:3 oder im Bereich 3:1:1 bis 1:1:3.

Durch diese gezielte Abmischung werden synergistische Effekte bezüglich der positiven Eigenschaften der kosmetischen Zubereitung generiert.

Als amphiphile Polymere und/oder assoziative Polymere (III) werden Polymere gewählt, die mindestens eine Fettsäure- oder Fettalkoholgruppe, als hydrohobe Gruppe, und eine hydrophile Gruppe tragen. Die Polymere sind wasserlöslich oder lassen sich als Mikrogele in Wasser dispergieren. Diese Polymere werden als quellbar bezeichnet. Die Polymere können jedweder chemischen Natur zusammengesetzt sein, z.B. Radikalpolymere, Vinyloder Acrylic-Polymere, Polykondensate und/oder deren Mischungen. Die Polymere können ionisch oder nichtionisch aufgebaut sein, wobei anionische und nichtionische Polymere bevorzugt sind.
Bevorzugt können als amphiphile bzw. assoziative Polymere gewählt werden:
Celluloseether enthaltend hydrophobe Substituenten, wie Alkylgrupen mit einer Kohlenstoff Anzahl gleich oder größer 8, wie beispielsweise Hydroxyethylcellulose Natrosol Plus Grade 330 der Firma Aqualon.
Quaternisierte kationische Cellulose mit mindestens einer Fettsäuregruppe, wie Alkyl-, Arylalkyl- oder Alkylaryl-Gruppe groups oder deren Mischungen, bevorzugt mit einer Kohlenstoffanzahl C8 -C22.
Quaternisierte Alkylhydroxyethylcellulose, wie beispielseise unter dem Namen Quatrisoft LM 200, Quatrisoft LM-X 529-18-A, Quatrisoft LM-X 529-18-B (C12 alkyl) und/oder Quatrisoft LM-X 529-8 (C18 alkyl) der Firma Amerchol erhältlich. Bevorzugt sind ebenso quaternisierte Alkylhydroxyethylcellulose, die unter dem Namen Crodacel QM, Crodacel QL (C12 alkyl) und/oder Crodacel QS (C18 alkyl) der Firma Croda erhältlich sind.
Galaktomannane enthaltend hydrohobe Substituenten, insbesondere die Derivate wie sie in EP-A-281360 offenbart sind.
Pullulane modifiziert durch hydrophobe Gruppen, insbesonder Cholesterolgruppen.
Gelatine modifiziert durch hydrophobe Gruppen, insbesondere C6 - C18 Alkylgruppen;
Mucopolysaccharide, erhaltend aus Glycosaminoglycane und Hyaluronicsäure.

Weiter bevorzugt können solche assoziative bzw. amphiphile Polymere eingesetzt werden, wie sie in EP-B1-1046387 offenbart werden, die hiermit explizit zum Offenbarungsgehalt der vorliegenden Erfindung zählen.

Als besonders geeignete assoziative Polymere haben sich solche herausgestellt, gewählt aus der Gruppe:
- der Polyethylenglycolether der allgemeinen Formel R-O-(-CH₂-CH₂-O-)ₙ-R', wobei R und R' unabhängig voneinander verzweigte oder unverzweigte Alkyl-, Aryl- oder Alkenylreste und n eine Zahl größer als 100 darstellen,
- der veretherten Fettsäureethoxylate der allgemeinen Formel R-COO-(-CH₂-CH₂-O-)ₙ -R', wobei R und R' unabhängig voneinander verzweigte oder unverzweigte Alkyl-, Aryl- oder Alkenylreste und n eine Zahl größer als 100 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel R-O-(-CH₂-CH(CH₃)-O-)ₙ-R', wobei R und R' unabhängig voneinander verzweigte oder unverzweigte Alkyl-, Aryl- oder Alkenylreste und n eine Zahl größer als 100 darstellen, der veresterten Fettsäurepropoxylate der allgemeinen Formel R-COO-(-CH₂-CH(CH₃)-O-)ₙ-C(O)-R', wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel R-O-Xₙ-Yₘ-R', wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist
- der veretherten Fettsäurepropoxylate der allgemeinen Formel R-COO-Xₙ-Yₘ-R', wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist
- Tetramethoxymethylglycoluril

Vorteilhafte erfindungsgemäße Polymere zeichnen sich insbesondere durch die folgende Strukturformel aus 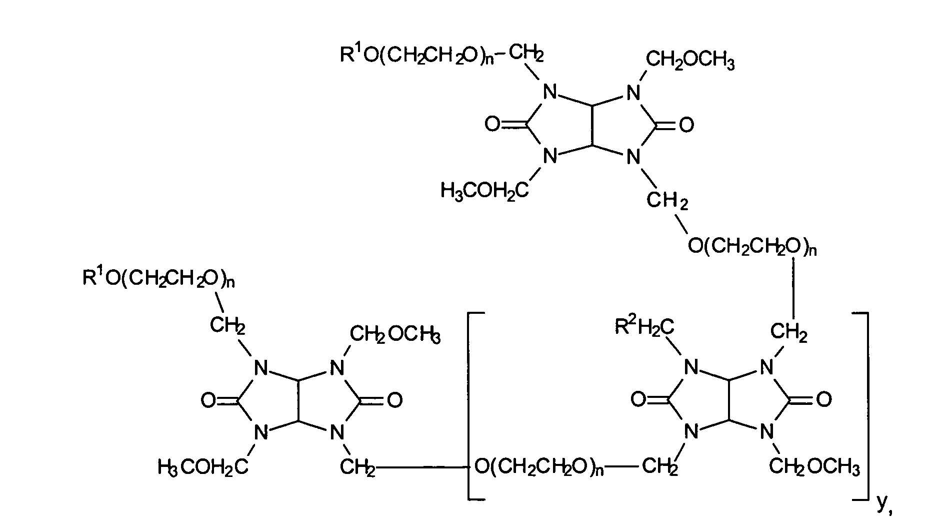 worin
R¹ einen verzweigten oder unverzweigten, gesättigten oder ungesättigten Alkylrest mit 4 bis 40 Kohlenstoffatomen,
R² = -OCH₃ oder -O(CH₂CH₂O)ₓR¹,
x eine ganze Zahl von 1 bis 100,
n eine ganze Zahl von 100 bis 250 und
y durchschnittlich 2 oder 3
bedeuten.

Besonders vorteilhafte erfindungsgemäße Polymere sind solche, für die n eine ganze Zahl von 150 bis 200 ist. Es ist insbesondere vorteilhaft im Sinne der vorliegenden Erfindung, wenn darüberhinaus R¹ einen verzweigten oder unverzweigten, gesättigten oder ungesättigten Alkylrest mit 8 bis 12 Kohlenstoffatomen darstellt.

Es ist ferner vorteilhaft im Sinne der vorliegenden Erfindung, wenn die mittlere Molmasse der Polymere zwischen 30.000 und 50.000 liegt.

Erfindungsgemäß ganz besonders vorteilhaft sind PEG-180/Octoxynol-40/Tetramethoxymethylglycoluril Copolymere mit R² = -O(CH₂CH₂O)₄₀C₈H₁₇ und n = 180.

Erfindungsgemäß ferner besonders vorteilhaft sind PEG-180/Laureth-50/Tetramethoxymethylglycoluril Copolymere mit R² = -O(CH₂CH₂O)₅₀C₁₂H₂₅ und n = 180.

Erfindungsgemäß ferner besonders vorteilhaft ist der Polyether-1.

Insbesondere vorteilhaft sind das PEG-150-Distearat und das PEG-150-Dioleat. Auch das PEG-300-Pentaerythrityltetraisostearat, das PEG-120 Methylglucosedioleat, das PEG-160-Sorbitantriisostearat, das PEG-450-Sorbitolhexaisostearat und das PEG-230-Glyceryltriisostearat sind vorteilhaft als assoziative Polymere zu verwenden.

Es ist demgemäß auch vorteilhaft hydrophob substituierte Polysaccharidderivate als assoziative Verdicker zu wählen, beispielsweise hydrophob substituierte Celluloseether, hydrophob substituierte Stärken, Alginate, Glucane, Chitine, Dextrane, Caseinate, Pektine, Proteine und Gummen, ferner Polyurethane, Polyacrylamide, Polyvinylalkohole, Polyacrylate und dergleichen mehr.
Insbesondere vorteilhaft sind die in der US-Patentschrift 5,426,182 beschriebenen hydrophob substituierten Polysaccharidderivate, die hiermit explizit zum Offenbarungsgehalt der vorliegenden Erfindung zählen.

Es kann auch gegebenenfalls vorteilhaft sein, wenn der oder die erfindungsgemäß verwendeten assoziativen Polymere über physiologische Wirksamkeit im Sinne einer kosmetischen oder pharmazeutischen Wirkung verfügt oder verfügen. So können beispielsweise die in DE-A1-4344661 offenbarten biotensiden Ester vorteilhaft im Sinne der vorliegenden Erfindung verwendet werden.

Als bevorzugt sind nichtionische Polymere wie Pure Thix TX -Typen, Crosspolymere wie Acrylates/Vinyl Isodecanoate Crosspolymer (Stabylen 30 von 3-V-Sigma) und Acrylate/C10-C30 Alkyl Acrylate Crosspolymer ( Pemulen TR 1, Pemulen TR 2, Ultrez 21, Carbopol ETD 2020, Carbopol ETD 2001 von Noveon), sowie hydrophob modifizierte Polyacrylate (HASE-Typen) wie Acrylates/Steareth-20 Methacrylate Copolymer (Acrysol bzw. Aculyn 22 von Rohm & Haas), Acrylates/ Steareth-20 Itaconate Copolymer (Structure 2000 v. National Starch) zu nennen.

Es ist vorteilhaft im Sinne der vorliegenden Erfindung, die Gesamtmenge der assoziativen bzw. amphiphilen Polymere aus dem Bereich von 0,01 bis 5 Gew.-%, vorteilhaft von 0,1 bis 1 Gew.-%, jeweils bezogen auf das Gesamtgewicht der Formulierung, zu wählen.

Die bevorzugten amphiphilen Polymere sind in der nachfolgenden Tabelle 1 aufgelistet, in der neben der Strukturformel und INCI Bezeichnung auch deren Handelsname aufgeführt ist.

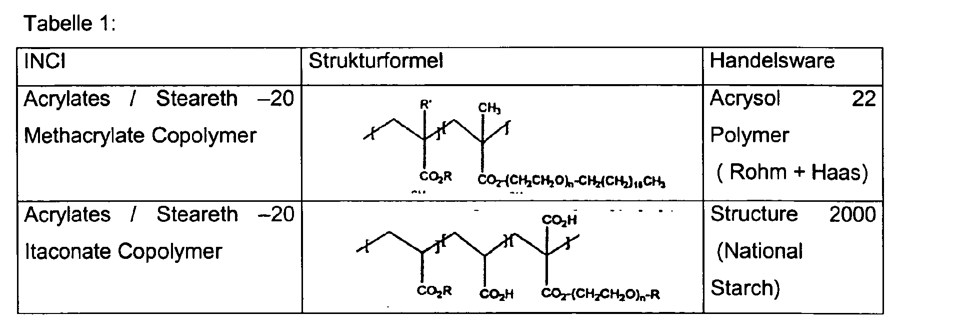

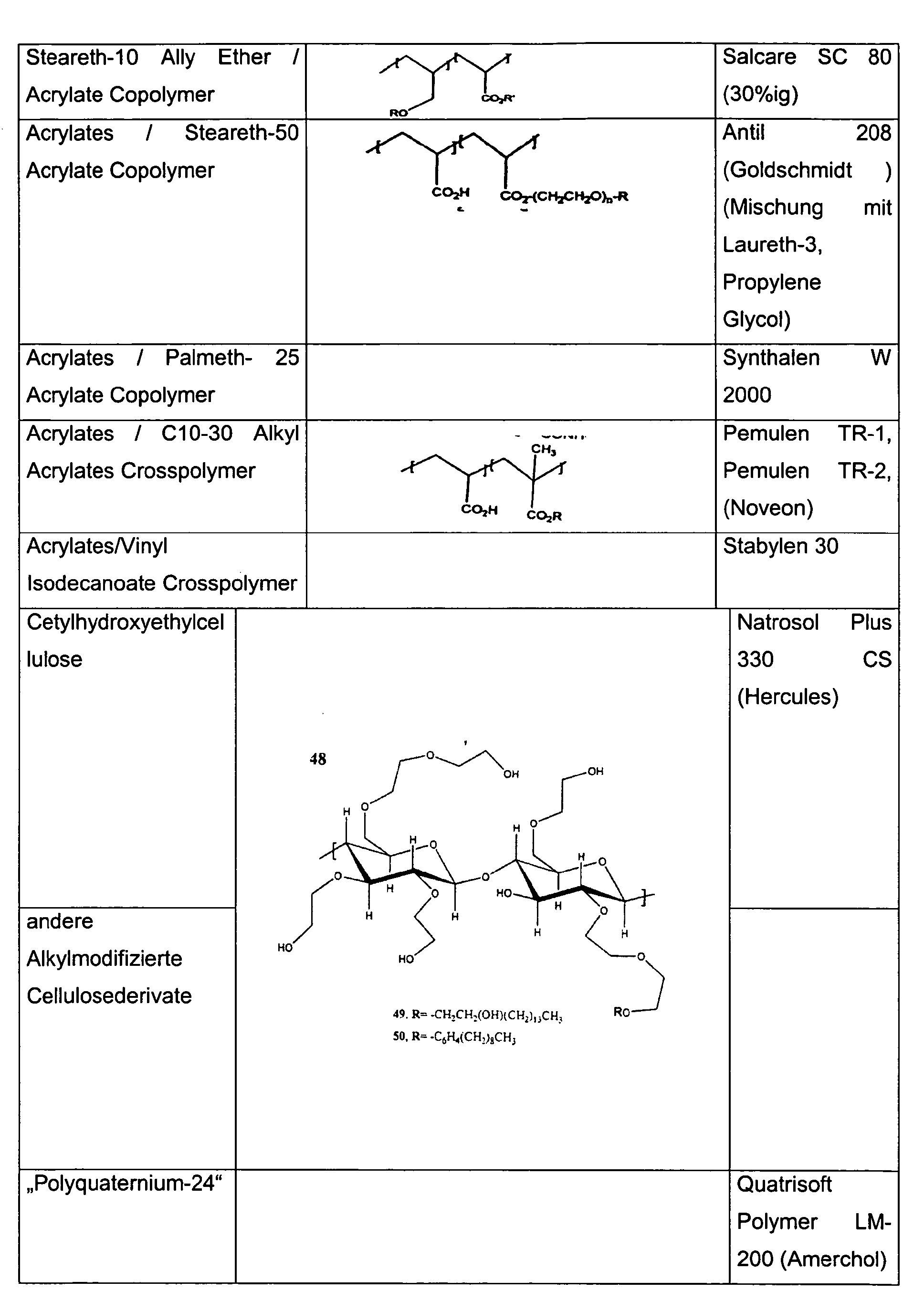

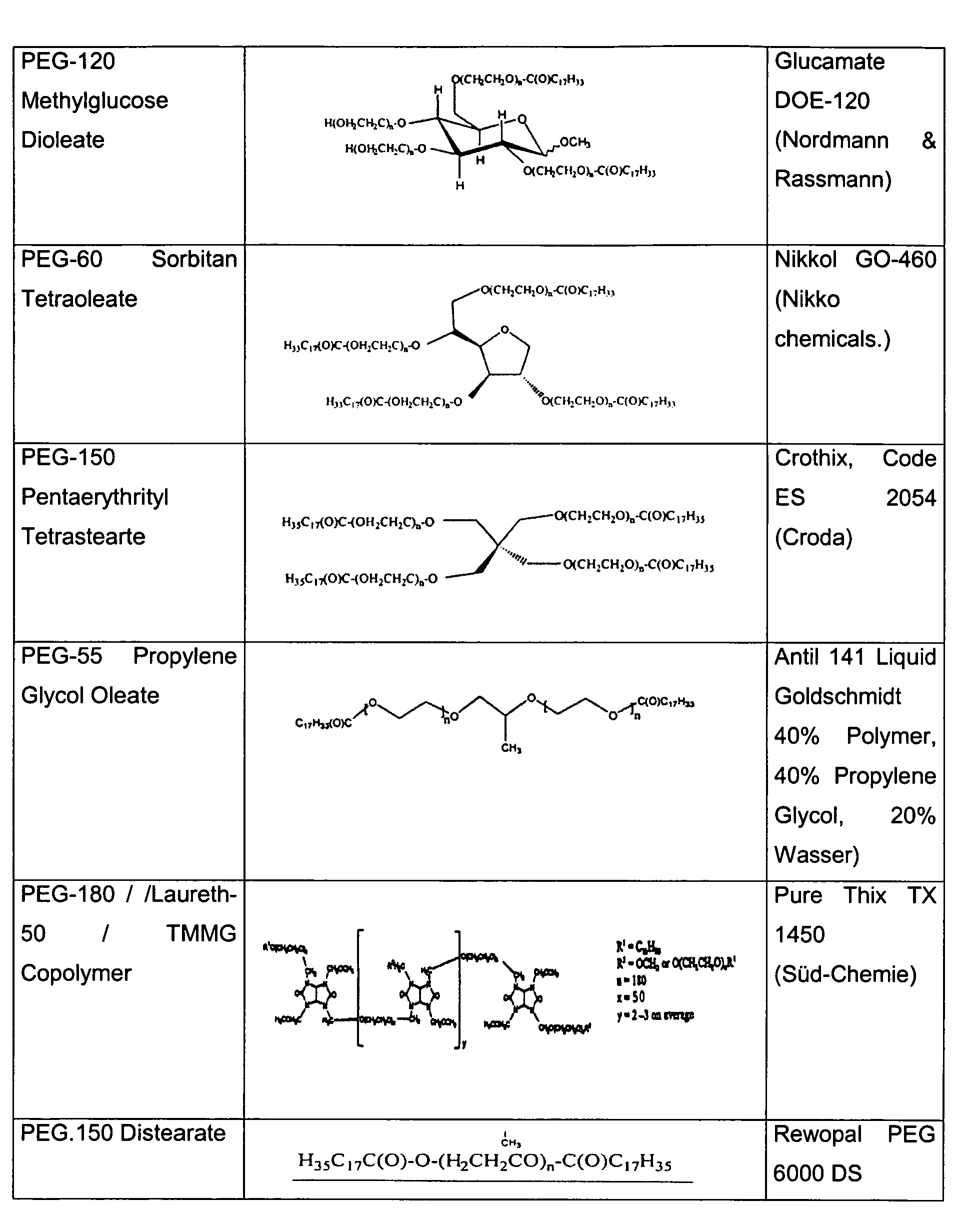

Der Einsatz von festen elastomeren Polyorganosiloxanen oder Organopolysiloxanen, im folgenden als Siloxanelastomere bezeichnet, in kosmetischen Zubereitungen ist an sich bekannt und hat in den letzten Jahren an Bedeutung gewonnen. Diese Stoffe fanden neben dem Einsatz in Kosmetika Verwendung in Lebens- und Futtermittel, Arzneimitteln, Imprägniermitteln, Schmiermitteln und so fort. Siloxalelastomere sind teilweise oder vollständig vernetzt und weisen zumeist eine dreidimensionale Struktur auf. Sie sind erhältlich durch eine Reaktion von vinyl-endständigem Polymethylsiloxan und Methylhydrodimenthylsiloxan oder auch durch Reaktion von Hydroxy-endständigem Dimethylpolysiloxan und Trimethylsiloxy-endständigem Methylpolysiloxan:

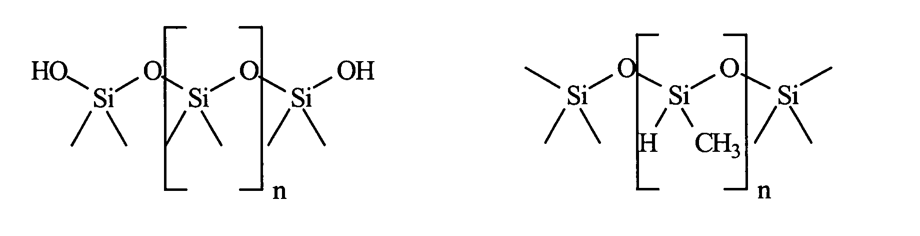

Diese Siloxanelastomere werden beispielsweise zur Einstellung der rheologischen Eigenschaften einer Zubereitung eingesetzt. Beschrieben werden derartige Siloxanelastomere beispielsweise in der europäischen Patentschrift 295886 sowie der US-Patentschrift 5266321, die die Verwendung dieser Stoffe in Gesichtsreinigungsmitteln beziehungsweise ölhaltigen Schminkprodukten offenbaren. In diesen Schriften ist auch die Natur der Siloxanelastomere näher beschrieben. Der Einsatz der Siloxanelastomere erfolgt in kosmetischen Zubereitungen insbesondere aufgrund ihrer angenehmen sensorischen Eigenschaften, die resultierenden Produkte werden als samtig, pudrig und/oder mattierend beschrieben. Daneben weisen sie stabilisierende Effekte auf hoch ölhaltige Formulierungen mit geringen Wassergehalten von höchstens 5 Gew.% auf. Bei der Formulierung der oben genannte Produkte stellt sich oftmals das Problem, das die Siloxanelastomere mit anderen häufig eingesetzten Komponenten unverträglich sind, was zu unbefriedigender Langzeitstabilität der Produkte führt.
Diese aus dem Stand der Technik bekannten Nachteile sind erfindungsgemäß überwunden worden.

Dabei ist es bevorzugt, wenn Siloxanelastomere gewählt aus den Gruppen der Siloxanelastomere, (a) die Einheiten R₂SiO und RSiO_{1,5} und/oder R3SiO_{0,5} und/oder SiO₂ enthalten, wobei die einzelnen Reste R jeweils unabhängig voneinander Wasserstoff, Alkyl wie beispielsweise Methy, Ethyl, Propyl oder Aryl wie beispielsweise Phenyl oder Tolyl, Alkenyl wie beispielsweise Vinyl bedeuten und das Gewichtsverhältnis der Einheiten R₂SiO zu RSiO_{1,5} im Bereich von 1:1 bis 30:1 gewählt wird; (b) die in Silikonöl unlöslich und quellfähig sind, die durch die Additionsreaktion eines Organopolysiloxans (1), das siliciumbebundenen Wasserstoff enthält mit einem Organopolysiloxan (2), das ungesättigte aliphatische Gruppen enthält, erhältlich sind, wobei die verwendeten Mengenateile so gewählt werden, daß die Menge des Wasserstoffes des Organopolysiloxans (1) oder der ungesättigten aliphatischen Gruppen des Organopolysiloxans (2) im Bereich von 1 bis 20 mol-% liegt, wenn das Organopolysiloxan nicht cyclisch ist und im Bereich von 1 bis 50 mol-% liegt, wenn das Organopolysiloxan cyclisch ist, verwendet werden. Besonders bevorzugt ist es, wenn das Organopolysiloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird. Ganz besonders bevorzugt ist es, wenn das Organopolysiloxanelastomer in Kombination mit unverzweigten bei Raumtemperatur flüssigen oder pastösen Silikonölen oder cyclischen Silikonölen oder deren Gemischen verwendet wird. Ganz außergewöhnlich bevorzugt ist es, wenn das Organopolysiloxanelastomer in Form eines Gels aus Organopolysiloxanelastomer und einer Lipidphase verwendet wird, wobei der Gehalt des Organopolysiloxanelastomers in dem Gel 3 bis 80 Gew.% , ganz außergewöhnlich stark bevorzugt 0,3 bis 60 Gew.% beträgt.

Es ist von Vorteil, in den erfindungsgemäßen Formulierungen Siloxanelastomere der Gesellschaft Dow Corning, wie sie in der US-amerikanischen Patentschrift 5654362 beschrieben und unter der Handelsbezeichnung 9040 Silicone Elastomer Blend erhältlich sind, einzusetzen. Ebenfalls vorteilhaft sind Siloxanelastomere der Gesellschaft Grant Chemical mit der INCI-Bezeichnung Polysilicone-11 wie die Typen Gransil GCM oder Gransil PM, wie sie in den US-amerikanischen Patentschriften 5266321, 4980167 und 4742142 beschrieben sind. Besonders vorteilhafte Siloxanelastomere sind weiterhin solche, die in Form sphärischer Puder mit einer durchschnittlichen Partikelgröße von 2 bis 5 µm und einer Partikelgrößenverteilung von 1 bis 15 µm vorliegen und in den japanischen Patentschriften 4-66446 und 4-17162 sowie in der japanischen Offenlegungsschrift 2-243612 beschrieben sind oder mit einer durchschnittlichen Partikelgröße von kleiner 50 µm in den Schriften EP 0295 886 und US 4761454 beschrieben sind. Kommerziell erhältliche Produkte sind beispielsweise Torayfil E-505C, Torayfil E-506 C von Toray-Dow Corning Silicone Co.
Ein besonders vorteilhaftes Siloxane Elastomer in Form von sphärischem Puder ist DimethiconeNinydimethicone Crosspolymer mit einer Partikelgrößenverteilung von 1 bis 15 µm, erhältlich unter der Handelsbezeichnung Dow Corning 9509 Powder von Dow Corning. Weiterhin vorteilhaft sind die Siloxanelastomere enthaltenden Gele, die unter den Handelsbezeichnungen KSG-15, -16, -17, -18, -20 von der Gesellschaft Shin-Etsu oder Gransil 5CYCgel, Gransil SR DMF 10gel, Gransil SR DC 556gel der Gesellschaft Grant Chemical, SF 839, SF1204, JK113 der Gesellschaft General Electric sowie Lauryl Dimeticon/Vinyldimethicon Crosspolymere, wie sie unter den Bezeichnungen KSG41, -42, - 43, -44 von der Gesellschaft Shin-Etsu erhältlich sind. Ein weiteres vorteilhaftes Siloxanelastomer ist das Cyclomethicon + Vinyldimethicon/Methicon Crosspolymer bzw. das chemisch verwandte Elastomer Crosslinked Stearyl Methyl Dimethyl Siloxane Elastomer , wie sie zum Beispiel von der Gesellschaft Grant Chemical unter der Bezeichnung SR-CYC angeboten werden.

Als Siloxanelastomere werden auch Emulsionen und/oder Suspensionen eingesetzt, die Siloxane Elastomere enthalten. Beispielhaft bevorzugt sind Emulsionen bzw. Suspensionen der nachfolgenden Tabelle 2.

**Tabelle 2:**

| **Handelsname/ BDF** | **INCI** | **Elastomergehalt** |
|---|---|---|
| Dow Corning 9040 Emulsion/ D Dow Corning 9040 Concentrated D Emulsion | Cyclopentasiloxane (and) Dimethicone Crosspolymer (and) Cyclohexasiloxane (and) Cyclomethicone (and) Dimethicone (and) Laureth-4 (and) Laureth-23 (and) Parabene *) | 70,6% DC 9040; 10,8% DC345; 5,4% DC 200; 1,9% Brij35; 0,8% Brij30; 0,55%Liquapar Optima; 9,95% Wasser |
| Dow Corning 9509 Silicone Elastomer Suspension/ Dow Corning 9509 | Dimethicone/ Vinyl Dimethicone Crosspolymer + C12 - 14 Pareth- 12 | 63% sphärische Siloxanelastomere in Wasser |

Die Siloxan-Elastomere (III) werden zu einem Anteil von bis zu 10 Gew.%, bevorzugt von 0,5-10 Gew.% des reinen Elastomers, bezogen auf die Gesamtzubereitung, eingesetzt.

Zusätzlich werden bevorzugt niedermolekulare Tenside (VI), insbesondere Ceteareth-3, Ceteareth-6, Steareth-2, Steareth-10, Ceteth-10, Isosteareth-10, Laureth-2, Laureth-3, Laureth-4, Myreth-4, Laneth-5, Ceteth-2, Ceteth-3, Oleth-2, Oleth-3 und/oder Oleth-5 den erfindungsgemäßen Zubereitungen zugesetzt. Ganz besonders bevorzugt ist Laureth-4.

Als besonders vorteilhafte Zubereitungen haben sich die folgenden Formulierungen ergeben, die umfassen
(I) bis zu 10 Gew.% Stearinsäure/Palmitinsäure,
(II) 0,1 - 10 Gew.% Cetyl Alkohol, Behenyl Alkohol, Stearyl Alcohol und/oder Cetearyl Alkohol
(III) 0,01 - 10 Gew.% Dimethicon/Vinyl Dimethicon Crosspolymer, Polysilicone -11, Acrylat/Alkyl Acrylat Cosspolymer, Acrylat/Vinyl Isodecanoat Crosspolymer, Acrylat/Steareth -20 Methacrylat Copolymer, Acrylat/Steareth -20 Itaconat Copolymer, Acrylat/Steareth-50 Acrylat Copolymer, Acrylat /Palmeth- 25 Acrylat Copolymer, Steareth-10 Ally Ether/Acrylat Copolymer, PEG-120 Methylglucose Dioleat, PEG-60 Sorbitan Tetraoleat, PEG-150 Pentaerythrityl Tetrasteart, PEG-55 Propylen Glycol Oleat, PEG-150 Distearat und/oder PEG-180 / Laureth-50 /TMMG Copolymer
(IV) 0,15 - 1 Gew.% Natronlauge
(V) bis zu 10 Gew.% PEG-20-Stearat, PEG-40-Stearat und/oder PEG-100 Stearat und
(VI) gegebenenfalls bis zu 10 Gew.% Steareth-2, Laureth-4 und/oder Ceteth-3

Ebenso haben sich Zubereitungen mit den folgenden Formulierungen als vorteilhaft erwiesen
(I) bis zu 12 Gew.% Stearinsäure/Palmitinsäure,
(II) 0 - 3 Gew:% Cetyl Alkohol, Behenyl Alkohol, Stearyl Alcohol und/oder Cetearyl Alkohol
(III) 0,01 - 10 Gew.% Dimethicon/Vinyl Dimethicon Crosspolymer, Polysilicone -11, Acrylat/Alkyl Acrylat Cosspolymer, Acrylat/Vinyl Isodecanoat Crosspolymer, Acrylat/Steareth -20 Methacrylat Copolymer, Acrylat/Steareth -20 Itaconat Copolymer, Acrylat/Steareth-50 Acrylat Copolymer, Acrylat /Palmeth- 25 Acrylat Copolymer, Steareth-10 Ally Ether/Acrylat Copolymer, PEG-120 Methylglucose Dioleat, PEG-60 Sorbitan Tetraoleat, PEG-150 Pentaerythrityl Tetrasteart, PEG-55 Propylen Glycol Oleat, PEG-150 Distearat und/oder PEG-180 / Laureth-50 /TMMG Copolymer
(IV) 0,25 - 1 Gew.% Natronlauge

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Vorteilhaft werden der oder die weiteren Emulgatoren aus der Gruppe gewählt, die die folgenden Verbindungen umfaßt:
Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, PEG-5-Soyasterol, PEG-6 Sorbitan Beeswax, Methylglucosesesquistearate, PEG-10 Hydrogenated Castor Oil, Sorbitanpalmitat, PEG-22/Dodecylglykol Copolymer, Polyglyceryl-2-PEG-4-Stearat, Sorbitanlaurat, PEG-4-Laurat, Polysorbat 61, Polysorbat 81, Polysorbat 65, Polysorbat 80, Triceteareth-4-Phosphat, Triceteareth-4 Phosphate und Sodium C₁₄₋₁₇ Alkyl Sec Sulfonat (Hostacerin CG von Hoechst), Polysorbat 85, Trilaureth-4-Phosphat, PEG-35 Castor Oil, Sucrosestearat, Trioleth-8-Phosphat, C₁₂₋₁₅ Pareth-12, PEG-40 Hydrogenated Castor Oil, PEG-16 Soya Sterol, Polysorbat 80, Polysorbat 20, Polyglyceryl-3-methylglucose Distearat, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, PEG-6 Caprylic/Capric Glycerides, Glyceryloleat und Propylenglykol, Polysorbat 60, Polyglyceryl-3 Oleat, PEG-40-Sorbitanperoleat, Laureth-4, Isostearylglycerylether, Cetearyl Alcohol und Natriumcetearylsulfat, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-Isostearat, PEG-40-Sorbitanperoleat, PEG-40-Sorbitanperisostearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, Glycerylstearatcitrat, Cetylphosphat, Polyglycerolmethylglucosedistearat, Poloxamer 101, Kaliumcetylphosphat, Polyglyceryl-3-Diisostearate und/oder AbilCare 85 von Dow Corning.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureestern des Sorbitols.

Die Gesamtmenge der weiteren Emulgatoren wird erfindungsgemäß vorteilhaft kleiner als 5 Gew.%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte, perlglänzende Zubereitungen im Sinne der vorliegenden Erfindung sind frei von Mono- und/oder Difettsäureester des Glycerin und/oder Glycols. Diese üblicherweise genutzten Emulgatoren werden erfindungsgemäß vorteilhafterweise nicht eingesetzt, um den erfindungsgemäßen Perlglanzeffekt zu gewährleisten.

Insbesondere bevorzugt sind erfindungsgemäße Zubereitungen, welche kein Glycerylstearat, Glycerylisostearat, Glyceryldiisostearat, Glyceryloleat, Glycerylpalmitat, Glycerylmyristat, Glyceryllanolat und/oder Glyceryllaurat enthalten.

Zusätzlich können in den Zubereitungen Lösungsvermittlern enthalten sein, wie beispielsweise PEG-40 Hydrogenated Castor Oil. Der Vorteil der Lösungsvermittler ist, dass sie die Perlglanzstrukturen bei höheren Temperaturen fördern und demzufolge einen zusätzlichen ästhetischen Nutzen generieren. Als geeignete Lösungsvermittler können gewählt werden Polysorbate 80, Polysorbate 60, PEG-40 Castor Oil, PEG-40 Hydrogenated Castor Oil, PEG-60 Hydrogenated Castor Oil, Polyglyceryl-3 Laurate, PEG-20 Glyceryl Laurate, Methyl Gluceth-20, Nonoxynol-10, PPG-1-PEG-9 Lauryl Glycol Ether, Ceteth-16, PEG-16 Soy Sterol, PEG-10 Soy Sterol, C12-15 Pareth-12, Nonoxynol-14, Octoxynol-16, PEG-20 Glyceryl Stearate, Sorbeth-30, PPG-26-Buteth-26 + PEG-40 Hydrogeanted Castor Oil, Tri C12-13 Alkyl Citrate, Polyglyceryl-2 Isostearate, Polyglyceryl-2 Diisostearate, PPG-15 Stearyl Ether, PEG-10 Olive Glycerides, PPG-3 Methyl Ether, PEG-2 Diethylhexanoate, C20-40 Pareth-40, PEG-60 Almond Glycerides und/oder PEG-6 Caprylic/Capric Glycerides.

Zusätzlich können in den kosmetischen Formulierungen unpolare Lipide, Mineralöle, Silikonöle und/oder Wachse bis zu 30 Gew.%, bezogen auf die Gesamtmasse der Zubereitung, enthalten sein.
Bevorzugt sind die unpolaren Lipide oder Wachse gewählt aus der Gruppe der unpolaren Kohlenwasserstoffen, hydriertes Polyisobuten, Cyclomethicone, Dimethicone, Methylpalmitat und/oder Dimethiconol Stearat.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächen-spannung gegen Wasser weniger als 20 mN/m beträgt als unpolar solche, deren Grenzflächenspannung gegen Wasser mehr als 30 mN/m beträgt. Lipide mit einer Grenzflächenspannung gegen Wasser zwischen 20 und 30 mN/m werden im allgemeinen als mittelpolar bezeichnet.

Besonders vorteilhafte mittelpolare Lipide im Sinne der vorliegenden Erfindung sind die im folgenden aufgelisteten Substanzen:

| Hersteller | Handelsname | INCI-Name | Polarität [mN/m] |
|---|---|---|---|
| Stearinerie Dubois Fils | DUB VCI 10 | Isodecyl Neopentanoate | 29,9 |
| ALZO (ROVI) | Dermol IHD | Isohexyldecanoate | 29,7 |
| ALZO (ROVI) | Dermol 108 | Isodecyl Octanoate | 29,6 |
| | Dihexyl Ether | Dihexyl Ether | 29,2 |
| ALZO (ROVI) | Dermol 109 | Isodecyl 3,5,5 Trimethyl Hexanoate | 29,1 |
| Henkel Cognis | Cetiol SN | Cetearyl Isononanoate | 28,6 |
| Unichema | Isopropylpalmitat | Isopropylpalmitat | 28,8 |
| Dow Corning | DC Fluid 345 | Cyclomethicone | 28,5 |
| Dow Corning | Dow Corning Fluid 244 | Cyclopolydimethylsiloxan | 28,5 |
| Nikko Chemicals Superior Jojoba Oil Gold | Jojobaöl Gold | | 26,2 |
| Wacker | Wacker AK 100 | Dimethicone | 26,9 |
| ALZO (ROVI) | Dermol 98 | 2- Ethylhexanosäure 3,5,5 Trimethylester | 26,2 |
| Dow Corning | Dow Corning Fluid 246 | Offen | 25,3 |
| Henkel Cognis | Eutanol G | Octyldodecanol | 24,8 |
| Condea Chemie | Isofol 16 | Hexyl Decanol | 24,3 |
| ALZO (ROVI) | Dermol 139 | Isotridecyl 3,5,5 Trimethylhexanonanoate | 24,5 |

| Hersteller | Handelsname | INCI-Name | Polarität |
|---|---|---|---|
| Henkel Cognis | Cetiol PGL | Hexyldecanol (+) Hexyl Decyl Laurate | 24,3 |
| | Cegesoft C24 | Octyl Palmitate | 23,1 |
| Gattefossé | M.O.D. | Octyldodeceyl Myristate | 22,1 |
| | Macadamia Nut Oil | | 22,1 |
| Bayer AG, Dow Corning | Silikonöl VP 1120 | Phenyl Trimethicone | 22,7 |
| CONDEA Chemie | Isocarb 12 | Butyl Octanoicacid | 22,1 |
| Henkel Cognis | Isopropylstearat | Isopropyl Stearate | 21,9 |
| WITCO, Goldschmidt | Finsolv TN | C12-15 Alkyl Benzoate | 21,8 |
| Dr. Straetmans | Dermofeel BGC | Butylene Glycol Caprylate/Caprate | 21,5 |
| Unichema Huels | Miglyol 812 | Caprylic/Capric Triglyceride | 21,3 |
| Trivent (über S. Black) | Trivent OCG | Tricaprylin | 20,2 |
| ALZO (ROVI) | Dermol 866 | PEG " Diethylhexanoate/ Diisononanoate/ Ethylhexyl Isononanoate | 20,1 |

Besonders bevorzugt sind unpolare Lipide. Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| Hersteller | Handelsname | INCI-Name | Polarität |
|---|---|---|---|
| Total SA | Ecolane 130 | Cycloparaffin | 49,1 |
| Neste PAO N.V. (Lief. Hansen & Rosenthal) | Nexbase 2006 FG | Polydecene | 46.7 |
| Chemische Fabrik Lehrte | Polysynlane | Hydrogenated Polyisobutene | 44.7 |
| Wacker | Wacker Silikonöl AK 50 | Polydimethylsiloxan | 46,5 |
| EC Erdölchemie (Lieferant Bayer AG) | Solvent ICH | Isohexadecane | 43.8 |
| DEA Mineralöl (Lief. Hansen & Rosenthal) Tudapetrol | Pionier 2076 | Mineral Oil | 43.7 |
| DEA Mineralöl (Lief. Hansen & Rosenthal) Tudapetrol | Pionier 6301 | Mineral Oil | 43.7 |
| Wacker | Wacker Silikonöl AK 35 | Polydimethylsiloxan | 42,4 |
| EC Erdölchemie GmbH | Isoeikosan | Isoeikosan | 41.9 |
| Wacker | Wacker Silikonöl AK 20 | Polydimethylsiloxan | 40,9 |
| Condea Chemie | Isofol 1212 Carbonat | | 40,3 |
| Gattefossé | Softcutol O | Ethoxydiglycol Oleate | 40,5 |
| Creaderm | Lipodermanol OL | Decyl Olivate | 40,3 |
| Henkel | Cetiol S | Dioctylcyclohexane | 39,0 |
| DEA Mineralöl (Lief. Hansen & Rosenthal) Tudapetrol | Pionier 2071 | Mineral Oil | 38.3 |
| WITCO BV | Hydrobrite 1000 PO | Paraffinum Liquidum | 37,6 |
| Goldschmidt | Tegosoft HP | Isocetyl Palmitate | 36,2 |
| Condea Chemie | Isofol Ester 1693 | | 33,5 |

| Hersteller | Handelsname | INCI-Name | Polarität |
|---|---|---|---|
| Condea Chemie | Isofol Ester 1260 | | 33,0 |
| Dow Corning | Dow Corning Fluid 245 | Cyclopentasiloxan | 32,3 |
| Unichema | Prisorine 2036 | Octyl Isostearate | 31.6 |
| Henkel Cognis | Cetiol CC | Dicaprylyl Carbonate | 31,7 |
| ALZO (ROVI) | Dermol 99 | Trimethylhexyl Isononanoate | 31,1 |
| ALZO (ROVI) | Dermol 89 | 2- Ethylhexyl Isononanoate | 31,0 |
| Henkel Cognis | Cetiol OE | Dicaprylyl Ether | 30,9 |
| | Dihexylcarbonat | Dihexyl Carbonate | 30,9 |
| Albemarle S.A. | Silkflo 366 NF | Polydecene | 30,1 |
| Unichema | Estol 1540 EHC | Octyl Cocoate | 30.0 |

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der Guerbetalkohole wie beispielsweise Octyldodecanol, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die in den Hauptansprüchen geforderten Bedingungen eingehalten werden.

Auch beliebige Abmischungen von Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1 C (C₁₈₋₃₆ - Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.
Besonders vorteilhafte Fett- und/oder Wachskomponenten sind chemisch modifzierte Wachse und synthetische Wachse, wie beispielsweise die unter den Handelsbezeichnungen Ultrasil IWS (Dimethiconol Stearat) bei Noveon sowie Estol 1503 (Methylpalmitat) bei Uniqema erhältlich.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden, sofern die in den Hauptansprüchen geforderten Bedingungen eingehalten werden.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt: 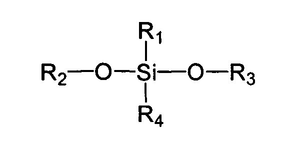 Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 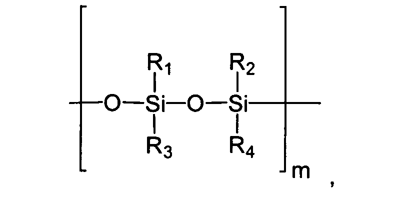 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 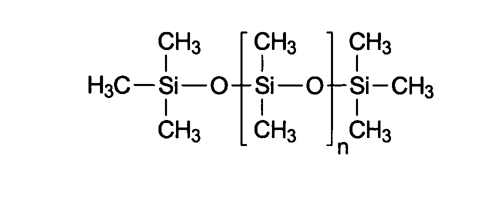 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| Hersteller | Handelsname | INCI-Name | Polarität [mN/m] |
|---|---|---|---|
| Wacker | Wacker Silikonöl AK 100 | Polydimethylsiloxan | 26,9 |
| Wacker | Wacker Silikonöl AK 50 | Polydimethylsiloxan | 46,5 |

| Hersteller | Handelsname | INCI-Name | Polarität |
|---|---|---|---|
| Wacker | Wacker Silikonöl AK 35 | Polydimethylsiloxan | 42,4 |
| Wacker | Wacker Silikonöl AK 20 | Polydimethylsiloxan | 40,9 |
| Dow Corning | Dow Corning Fluid 245 | Cyclopentasiloxan | 32,3 |
| Dow Corning | Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 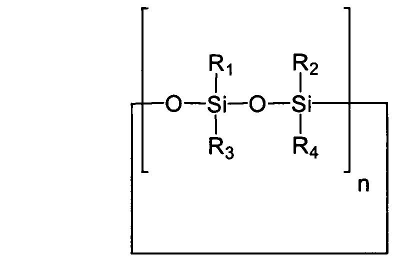 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Darüber hinaus können die Zubereitungen Lichtfilter, Farbstoffe, Wirkstoffe, Moisturizer, Puderrohstoffe, Füllstoffe wie Talkum, Silika, Bornitrid und Stärkederivate, Konservierungsmittel und/oder Deodorantien enthalten.

Es ist daher vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Lichtschutzfilter Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form commerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.
Die erfindungsgemäßen Titandioxide zeichnen sich durch eine Primärpartikelgröße zwischen 10 nm bis 150 nm aus.

| Handelsname | Coating | zusätzliche Bestandteile der Vordispersion | Hersteller |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica | - | Tayca Corporation |
| | Silikon | | |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure - | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 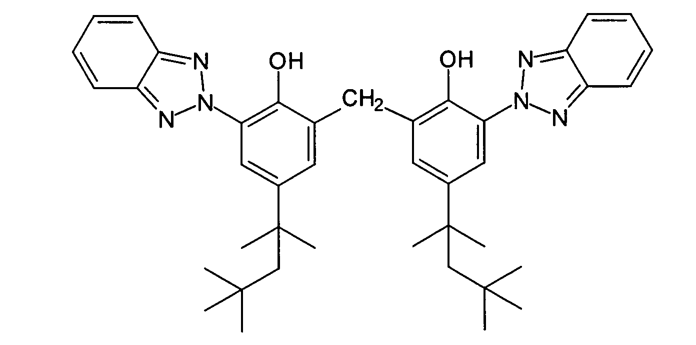 gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 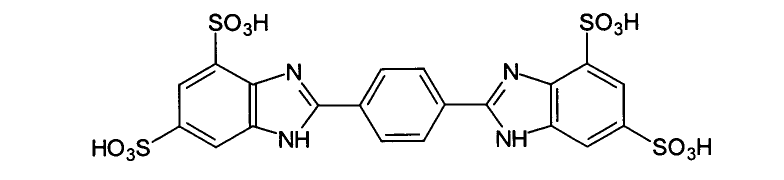 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 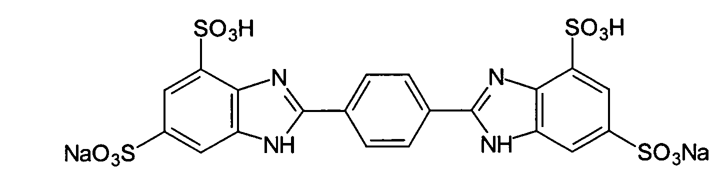 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

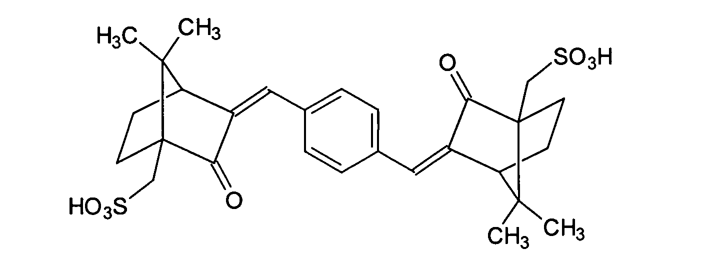

Weitere vorteilhafte UV-A-Filtersubstanzen sind Hydroxybenzophenone, die sich durch die folgende Strukturformel auszeichnen: 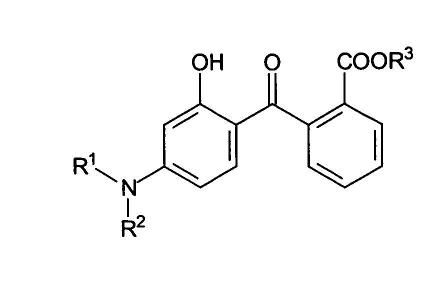 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 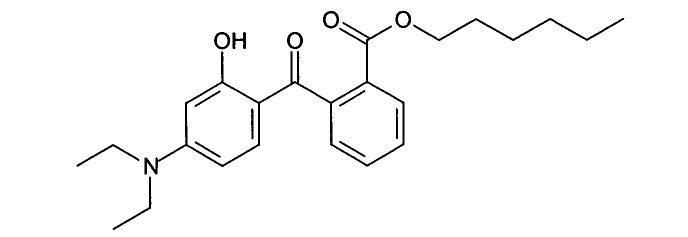 und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 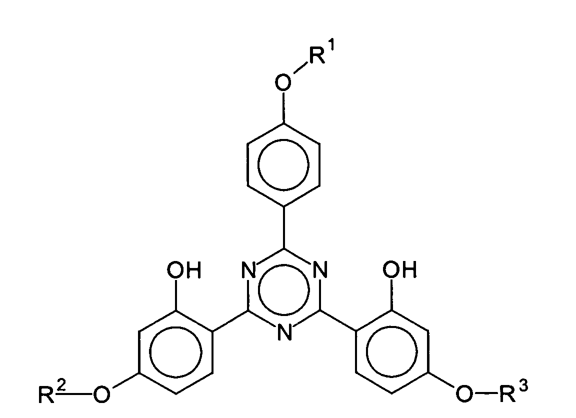 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{(4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 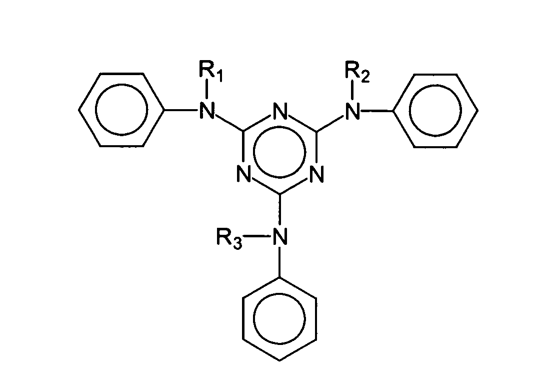 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 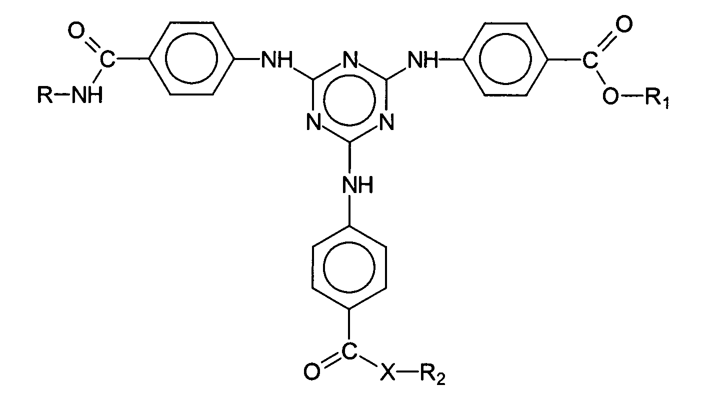 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 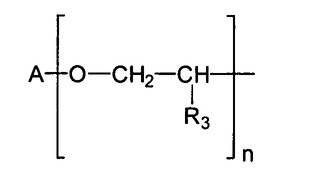 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 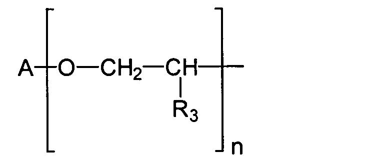 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 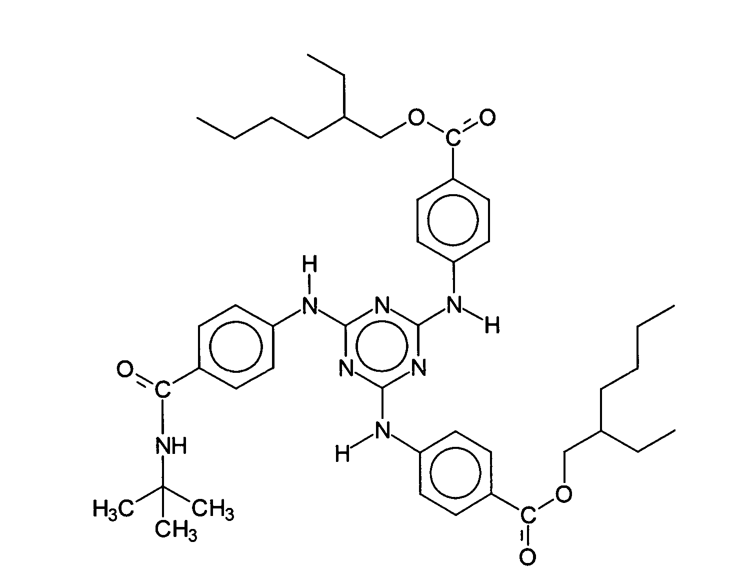 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoäsäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 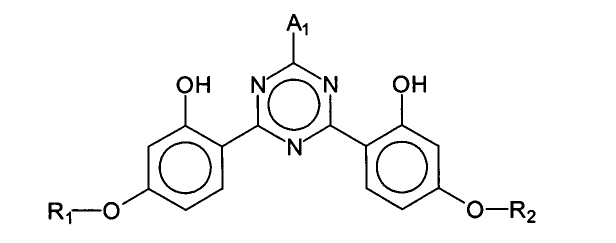 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexy)oxy)-2-hydroxy]-pheny)}-6-(1 -methyl-pyrrol-2-yl)-1,3,5-triazin, das 2,4-Bis-{[4-tris(trimethylsiloxy-sifylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin. Ein vorteilhafter Breitbandfilter im Sinne der vorliegenden Erfindung ist das 2,2'-Methylenbis-(6-(2H-benzotriazol-2-yl)-4-(1,1,3,3-tetramethylbutyl)-phenol), welches durch die chemische Strukturformel 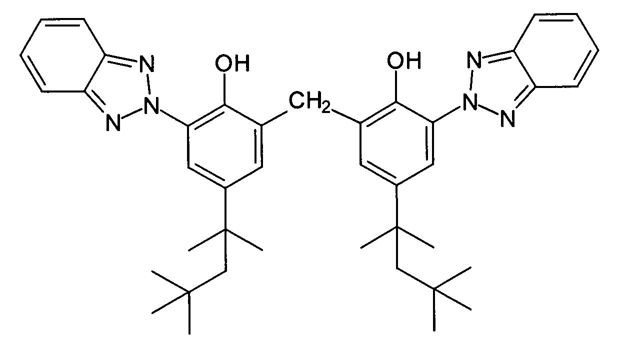 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1, 3,3,3-tetramethyl-1-[(trimethylsilyl)oxy]d i-siloxanyl]propyl]-phenol (CAS-Nr.: 155633-54-8) mit der INCI-Bezeichnung Drometrizole Trisiloxane, welches durch die chemische Strukturformel 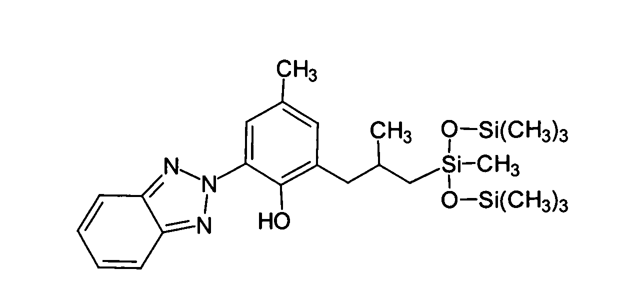 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

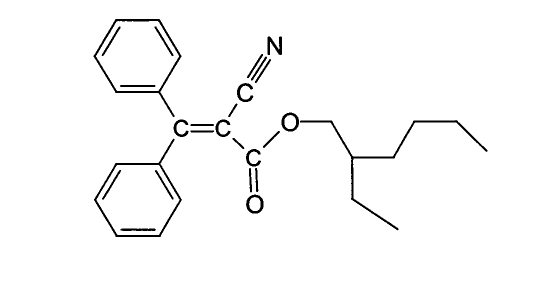

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

So betrifft die vorliegende Erfindung als besondere Ausführungsformen kosmetische und dermatologische Hautpflege- und/oder Lichtschutzzubereitungen, insbesondere hautpflegende oder dekorierende kosmetische und dermatologische Lichtschutzzubereitungen mit Perlglanzeffekt.

Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Ebenso lassen sich Moisturizer in die kosmetische Zubereitung einmischen. Als Hautbefeuchtungsmittel lassen sich vorteilhaft Glycerin, Chitosan, Fucogel, Propylenglycol, Dipropylenglycol, Butylenglycol, Mannitol, Milchsäure, Natriumpyrolidoncarbonsäure, Hyaluronsäure, Salze der angegebenen Säuren sowie Glycin, Harnstoff und Salze von Metallen der ersten und zweiten Hauptgruppe verwenden.

Besonders geeignet sind Glycerin, Milchsäure, Butylenglycol, Harnstoff, Hyaluronsäure.

Der Gehalt an Hautbefeuchtungsmitteln beträgt vorteilhaft 3 Gew.-% bis 60 Gew.-%, vorzugsweise 4 bis 50 Gew.-%, insbesondere 5 bis 40 Gew.-%, bezogen auf das Gesamtgewicht der Zubereitungen.

Erfindungsgemäße Zubereitungen können vorteilhaft auch Puder enthalten. Puder sind aus einer oder mehreren Pudergrundlagen zusammengesetzte, mehr oder weniger feinteilige, pulverförmige Zubereitungen, welchen, ihrem Anwendungszweck entsprechend, ein oder mehrere Wirkstoffe, Konservierungsmittel, Parfümöle, Farbstoffe usw. beigemischt sein können.

Das FDA's OTC Miscellaneous External Panel hat für Puder nachfolgende Definition festgelegt: "Eine homogene Dispersion feinzerteilten, relativ trockenen feinteiligen Materials, welches aus einer oder mehreren Substanzen besteht" (FDC Reports [Pink Sheet] 41, Nr. 33, T&G-4 [Aug. 13, 1979]).

Die Zusammensetzung eines Puders hängt weitgehend von den Aufgaben ab, die er zu erfüllen hat. Puder können aber auch Verdünnungsmittel für Medikamente, z. B. Antibiotika, Sulfonamide usw., sein. Flüssige Puder sind meist aus Talkum, Zinkoxid und/oder Titandioxid, Glycerin und Wasser bestehende dickflüssige Zubereitungen (Schüttelmixturen). Kompaktpuder sind durch hohen Druck brikettierte oder durch Zugabe von Calciumsulfat (Gips) zusammengesinterte Pudergrundlagen.

Zusätzlich werden Puder auch in Aerosolform zur Verfügung gestellt und angewandt, nachdem es gelungen ist, Ventile zu entwickeln, die die Möglichkeit einer Verstopfung der Ventilausführungsgänge weitgehend ausschließen.

Das immer zu befürchtende Sedimentieren der eingearbeiteten Puderteilchen kann ebenfalls verhindert werden, wenn man in die Rezeptur geeignete Suspendiermittel bzw. Suspendierhilfen einarbeitet, beispielsweise Alkalimetall-, Ammonium- oder Aminsalze eines Dialkylsulfosuccinats mit Alkylgruppen von 4-12 C-Atomen, z. B. Natriumdioctylsulfosuccinat (typischerweise ca. 0,002-0,015 Gew.-%), oder eine Alkylbenzolsulfonsäure mit Alkylgruppen von 8-14 C-Atomen, z. B. Natriumdodecylbenzolsulfonat.

In die erfindungsgemäßen Zubereitungen lassen sich je nach Anwendungsgebiet übliche kosmetische Füllstoffe, Zusatzmittel, Pigmente, Farbstoffe, Parfum und auch Pflege- und Wirkstoffe einarbeiten. In erfindungsgemäße Rezepturen lassen sich sowohl große Mengen hydrophiler, als auch hydrophober Wirkstoffe oder Kombinationen aus hydrophilen und hydrophoben Wirkstoffen in die Formulierungen einarbeiten. Derartige erfindungsgemäß vorteilhafte Wirkstoffe sind beispielsweise Acetylsalicylsäure, Azulen, Ascorbinsäure (Vitamin C), Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Zusätzlich können Pflegewirkstoffe eingearbeitet werden, welche sich nicht auf die fettlöslichen Wirkstoffe beschränken, sondern auch aus der Gruppe der wasserlöslichen Wirkstoffe gewählt werden können, beispielsweise Vitamine und dergleichen mehr.

Eine erstaunliche Eigenschaft der erfindungsgemäße Zubereitungen ist, dass diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeignet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Antihistaminika, Antiphlogistika, Antibiotika, Antimykotika, die Durchblutung fördernde Wirkstoffe, Keratolytika, Hormone, Steroide, Vitamine, Hormone, Steroide, Vitamine usw.

Durch den Einsatz amphiphiler Polymere und/oder assoziativer Polymere und/oder Siloxane Elastomere (III) ist es jetzt erstmals möglich, in Stearatesystemen auch bei Neutralisation mit NaOH als alleinige Base langzeitstabile Emulsionen mit kosmetischer Perlglanzoptik zu formulieren, die eine deutlich verbesserte Hautverträglichkeit aufweisen.

Durch die erfindungsgemäßen Zubereitungen ist es zudem erstmals möglich Perlglanz-Emulsionen zu formulieren, die
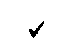 einen hohen Anteil an Lipiden, auch unterschiedlicher Polarität und Wachse bis zu einem Anteil von 30 Gew.% aufweisen,
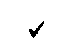 langzeitstabil sind,
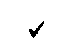 die sensorischen Eigenschaften der bisher sehr stumpf und klebrigen Perlglanzsysteme deutlich verbessern,
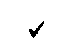 geschmeidig, weich, nicht klebrig, kosmetisch ausgleitende Eigenschaften aufweisen,
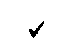 einen Fettsäuregehalt von weniger als 12 Gew.% mit einem verseiften Anteil von max. 9 Gew.% aufweisen,
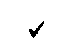 eine deutlich verbesserte Hautverträglichkeit aufweisen
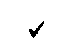 einen erhöhten Fettalkoholgehalt von bis zu 10 Gew.% enthalten können, ohne die Kristallbildung und damit die Perlglanzoptik zu beeinträchtigen

Eine bevorzugte Möglichkeit der Bildung von erfindungsgemäßen Emulsionen besteht darin, die Öltröpfchen durch den Einsatz hydrophob modifizierter, synthetischer oder natürlicher Polymere zu immobilisieren. Solche Polymere werden gelegentlich auch als assoziative Verdicker bezeichnet. Unter assoziative Polymere fallen Vernetzersubstanzen, im Rahmen der vorliegenden Beschreibung auch als Verdicker bezeichnet, die ein eigenständiges Gelnetzwerk bilden, in welchem die Emulsionströpfchen dann durch hydrophobe Wechselwirkung festgehalten werden. Somit liegen dann sogenannte assoziative bzw. amphiphile Verdicker vor. Der Zusammenhalt des Netzes kann dabei auch durch die Vernetzung mit den Emulsionströpfchen in den Knotenpunkten des Netzes bewirkt werden.

Gegenstand der Erfindung ist ferner die Verwendung der Zubereitung als Kosmetika zur Erzielung eines optisch ansprechenden Perlglanzeffektes. Die Zubereitungen können als Creme, Lotion, Schäume, Spray eingesetzt und dargereicht werden. Darüber hinaus als können die erfindungsgemäßen Zubereitungen als dekorierende Kosmetik, Make-up, Whitening-Produkte, Cooling-Produkte, Sonnenschutzmittel und vor allem als Gesichts- , Körper- und Handpflegeprodukte eingesetzt werden.

### Beispiele

| Beispiele | 1 | 2 | 3 | 4 | 5 | 6 | 7 | 8 |
|---|---|---|---|---|---|---|---|---|
| Stearinsäure / Palmitinsäure | 6,0 | 4,5 | 7,5 | 5,0 | 7,5 | 4,5 | 5,0 | 4,5 |
| PEG-20-Stearat | | 1,5 | | | | | 0,5 | |
| PEG-40-Stearat | 2,0 | | | 0,5 | 1,5 | 1,5 | | 4,5 |
| PEG-100 Stearat | | | 2,5 | 1,0 | 1,0 | | 0,5 | |
| Steareth-2 | | 0,5 | | | 0,5 | | 0,6 | |
| Laureth-4 | 1,0 | | | 0,5 | | 0,6 | 0,2 | 0,5 |
| Ceteth-4 | | | 0,2 | | | 0,4 | | |
| Cetyl Alkohol | 2,0 | | | 1,5 | | | 1,0 | |
| Behenyl Alkohol | | | 1,5 | | | | | |
| Stearyl Alcohol | | | 1,0 | | 2,5 | 0,5 | | |
| Cetearyl Alkohol | | 1,5 | | | | 1,0 | | 1,5 |
| Cetyl Palmitat | | | | 1,0 | | | | |
| Myristyl Myristat | | 1,0 | | | | | 1,0 | |
| Dimethiconol Stearat | 2,0 | | | | 4,0 | | | |
| Hydriertes Coco-Glycerid | | | 1,0 | | 0,5 | | | |
| Shea Butter | | 1,0 | | | | | 0,5 | |
| Silikonwachse (z.B. Abil Wax 9840) | | | 2,0 | | | | | |
| Methyl Palmitat | | | | 1,0 | | | 3,0 | |
| C12-15 Alkyl Benzoat | | | 3,0 | 3,0 | | | | |
| Butylen Glycol Dicaprylat/Dicaprat | | | | 1,0 | | | | |
| Caprylic/Capric Triglycerid | | | | | | | | 1,0 |
| Ethylhexylcocoat | | | | | | | | 2,0 |
| Octyldodecanol | | 3,0 | | | | | 3,0 | |
| Mineral Oil | | 2,0 | 1,0 | | | | | |
| Hydriertes Polyisobuten | 5,0 | | | | | 4,0 | | |
| Polydecen | | 2,0 | | | 2,0 | | 1,0 | |
| Petrolatum | | | 2,0 | | 2,0 | | | |
| Cyclomethicon | 5,0 | 2,0 | 1,0 | 2,0 | | 4,0 | | 8,0 |
| Dimethicon | 1,0 | 3,0 | | 2,0 | 2,0 | 2,0 | 1,0 | 2,0 |
| Phenyltrimethicon | | | 1,0 | | | 1,0 | | |
| Dicaprylyl Ether | | | 2,5 | | | | | |
| Dicarprylyl Carbonat | | | | | 3,0 | | | |
| Natürliche Öle (z.B. Triglyceride wie Jojobaöl) | 0,5 | | | 1,0 | | | | |
| Siloxane Elastomere (wie z.B. Dimethicon / Vinyl Dimethicon Crosspolymer; Dow Corning 9506 Powder oder Polysilicone -11, Gransil GCM-5) | | 1,0 | 2,0 | | 4,0 | | | |
| Hydrophob modifizierte Acrylate - Amphiphile Polymere wie z.B-Acrylat/Alkyl Acrylat Cosspolymer, Acrylat/Vinyl Isodecanoat Crosspolymer | 0,5 | 0,5 | | 0,25 | | 0,5 | | 0,5 |
| Assoziative Polymere vom HASE-Typ, (wie z.B. Acrylat/ Steareth -20 Methacrylat Copolymer, Acrylat/ Steareth -20 Itaconat Copolymer, Acrylat/ Steareth-50 Acrylat Copolymer, Acrylat / Palmeth-25 Acrylat Copolymer, Steareth-10 Ally Ether / Acrylat Copolymer) | | | 0,5 | | | 0,1 | | |
| Assoziative Polymere vom Typ Hydrophob modifizierte POE-Copolymere, (wie z.B. PEG-120 Methylglucose Dioleat, PEG-60 Sorbitan Tetraoleat, PEG-150 Pentaerythrityl Tetrasteart, PEG-55 Propylen Glycol Oleat, PEG-150 Distearat, PEG-180 / Laureth-50 / TMMG Copolymer) | | 0,1 | 0,1 | 0,25 | 0,5 | | 0,5 | |
| Trisodium EDTA | 0,2 | 0,1 | | 0,05 | | 0,1 | 0,3 | |
| Iminodisuccinat | 0,1 | | 0,1 | | 0,3 | | | 0,5 |
| Phenoxyethanol | 0,3 | 0,1 | | 0,5 | 0,8 | | | 0,4 |
| Parabene | 0,6 | | 0,4 | | 0,4 | | 0,2 | |
| Hexamidin Diisethionat | | 0,1 | | | 0,05 | | 0,1 | |
| Imidodiazolydynl Urea | | | | | | 0,2 | | 0,2 |
| DMDM Hydantoin | | | 0,2 | | | 0,1 | | |
| lodopropynyl Butylcarbamat | | | | 0,2 | | | 0,05 | |
| Alcohol Denat. | 5,0 | | 2,0 | | | 10,0 | | 8,0 |
| Octoxyglycerin > | | 1,0 | | | 3,0 | | 5,0 | |
| Xanthan Gum | | | | | 0,1 | | | |
| Carbomer | 0,05 | | | | | 0,2 | | |
| Polyacrylamid | | | 0,2 | | | | | 0,2 |
| Celluloseether | | | | | | | 0,1 | |
| C18-36 Acid Triglycerid | | | | 0,2 | | | | |
| PVP / Hexadecen Copolymer | | | | 0,1 | | | | |
| Tricontayl PVP | | | | | | 0,1 | | |
| Hydroxypropylcellulose | | | 0,05 | | | | | |
| Phenylbenzimidazole Sulfonic Acid | | | | | | | 2,0 | |
| Bis-Ethylhexyloxyphenol Methoxyphenyl Triazin | | | | 1,0 | | | | |
| Ethylhexyl Triazon | | | | 0,5 | | | | |
| Butyl Methoxydibenzoylmethane | | | | 1,0 | | | | |
| Disodium Phenyl Dibenzimidazole Tetrasulfonate | | | | | | | 1,0 | |
| Titandioxid T 805 | | | 0,50 | | | 0,50 | 0,50 | |
| Ethylhexyl Methoxycinnamat | | | | 2,0 | | | 4,0 | |
| Octocrylene | | | 1,5 | | | | | |
| Benzophenone-3 | | | 1,5 | | | | | |
| Füllstoffe (Distarch Phosphat, Tapiokastärke, Aluminium Starch Octenyl Succinat, Silica, Talcum, Boronnitrid) | | 2,0 | 4,0 | | | 3,0 | | |
| Parfuem | q.s. | q.s. | q.s. | q.s. | q.s. | q.s. | q.s. | q.s. |
| Natron- und/oder Kalilauge | q.s. | q.s. | q.s. | q.s. | q.s. | q.s. | q.s. | q.s. |
| Aqua | Ad 100 | Ad 100 | Ad 100 | Ad 100 | Ad 100 | Ad 100 | Ad 100 | Ad 100 |
| | | | | | | | | |
| Beispiele | 9 | 10 | 11 | 12 | 13 | 14 | 15 | 16 |
| Stearinsäure / Palmitinsäure | 10,0 | 10,0 | 7,5 | 6,0 | 10,0 | 8,5 | 10,0 | 10,0 |
| Cetyl Alkohol | 0,5 | | | | | | 0,5 | |
| Behenyl Alkohol | | | 0,5 | | | | | |
| Stearyl Alcohol | | | | | 0,25 | | | |
| Cetearyl Alkohol | | 0,25 | | | | | | 0,5 |
| Cetyl Palmitat | | | | 1,0 | | | | |
| Myristyl Myristat | | 1,0 | | | | | | |
| Dimethiconol Stearat | 1,0 | | | | | | | |
| Hydriertes Coco-Glycerid | | | 1,0 | | 0,5 | | | |
| Shea Butter | | 1,0 | | | | | 0,5 | |
| Silikonwachse (z.B. Abil Wax 9840) | | | 2,0 | | | | | |
| Methyl Palmitat | 5,0 | 10,0 | 20,0 | 5,0 | 5,0 | 10,0 | 10,0 | 5,0 |
| C12-15 Alkyl Benzoat | | | 3,0 | 3,0 | | | | |
| Butylen Glycol Dicaprylat/Dicaprat | | | | 1,0 | | | | |
| Caprylic/Capric Triglycerid | | | | | | | | 1,0 |
| Ethylhexylcocoat | | | | | | | | 2,0 |
| Octyldodecanol | | 3,0 | | | | | 3,0 | |
| Mineral Oil | | | 1,0 | | | | | |
| Hydriertes Polyisobuten | | | | | | 2,5 | | |
| Polydecen | | | | | 2,0 | | 1,0 | |
| Petrolatum | | | 2,0 | | 2,0 | | | |
| Cyclomethicon | 5,0 | 2,0 | 1,0 | 2,0 | | 4,0 | 5,0 | 8,0 |
| Dimethicon | 1,0 | 3,0 | | 2,0 | 5,0 | 2,0 | 1,0 | 2,0 |
| Phenyltrimethicon | | | 1,0 | | | 1,0 | | |
| Dicaprylyl Ether | | | 1,0 | | | | | |
| Dicarprylyl Carbonat | | | | | 3,0 | | | |
| Natürliche Öle (z.B. Triglyceride wie Jojobaöl) | 0,5 | | | 1,0 | | | | |
| Siloxane Elastomere (wie z.B. Dimethicon / Vinyl Dimethicon Crosspolymer; Dow Corning 9506 Powder oder Polysilicone -11, Gransil GCM-5) | | | 2,0 | | 4,0 | | | |
| Hydrophob modifizierte Acrylate - Amphiphile Polymere wie z.B-Acrylat/Alkyl Acrylat Cosspolymer, Acrylat/Vinyl Isodecanoat Crosspolymer | | | | | | 0,1 | 0,1 | |
| Assoziative Polymere vom HASE-Typ, (wie z.B. Acrylat/ Steareth -20 Methacrylat Copolymer, Acrylat/ Steareth - 20 Itaconat Copolymer, Acrylat/ Steareth-50 Acrylat Copolymer, Acrylat / Palmeth- 25 Acrylat Copolymer, Steareth-10 Ally Ether / Acrylat Copolymer) | 0,5 | 0,5 | 0,5 | 0,25 | | 0,5 | | 0,5 |
| Assoziative Polymere vom Typ Hydrophob modifizierte POE-Copolymere, (wie z.B. PEG-120 Methylglucose Dioleat, PEG-60 Sorbitan Tetraoleat, PEG-150 Pentaerythrityl Tetrasteart, PEG-55 Propylen Glycol Oleat, PEG-150 Distearat, PEG-180 / Laureth-50 / TMMG Copolymer) | | 0,1 | 0,1 | 0,25 | 0,5 | | 0,5 | |
| Trisodium EDTA | 0,2 | 0,1 | | 0,05 | | 0,1 | 0,3 | |
| Iminodisuccinat | 0,1 | | 0,1 | | 0,3 | | | 0,5 |
| Phenoxyethanol | 0,3 | 0,1 | | 0,5 | 0,8 | | | 0,4 |
| Parabene | 0,6 | | 0,4 | | 0,4 | | 0,2 | |
| Hexamidin Diisethionat | | 0,1 | | | 0,05 | | 0,1 | |
| Imidodiazolydynl Urea | | | | | | 0,2 | | 0,2 |
| DMDM Hydantoin | | | 0,2 | | | 0,1 | | |
| lodopropynyl Butylcarbamat | | | | 0,2 | | | 0,05 | |
| Alcohol Denat. | 5,0 | | 2,0 | | | 10,0 | | 8,0 |
| Octoxyglycerin | | 1,0 | | | 3,0 | | 5,0 | |
| Xanthan Gum | | | | | 0,1 | | | |
| Carbomer | 0,05 | | | | | 0,2 | | |
| Polyacrylamid | | | 0,2 | | | | | 0,2 |
| Celluloseether | | | | | | | 0,1 | |
| C18-36 Acid Triglycerid | | | | 0,2 | | | | |
| PVP / Hexadecen Copolymer | | | | 0,1 | | | | |
| Tricontayl PVP | | | | | | 0,1 | | |
| Hydroxypropylcellulose | | | 0,05 | | | | | |
| Phenylbenzimidazole Sulfonic Acid | | | | | | | 1,0 | |
| Bis-Ethylhexyloxyphenol Methoxyphenyl Triazin | | | | 1,0 | | | | |
| Ethylhexyl Triazon | | | | 0,5 | | | | |
| Butyl Methoxydibenzoylmethane | | | | 1,0 | | | | |
| Disodium Phenyl Dibenzimidazole Tetrasulfonate | | | | | | | 1,0 | |
| Titandioxid T 805 | | | 1,0 | | | 0,50 | 1,0 | |
| Ethylhexyl Methoxycinnamat | | | | 2,0 | | | 4,0 | |
| Octocrylene | | | 2,0 | | | | | |
| Benzophenone-3 | | | 2,0 | | | | | |
| Füllstoffe (Distarch Phosphat, Tapiokastärke, Aluminium Starch Octenyl Succinat , Silica, Talcum, Boronnitrid) | | 2,0 | 4,0 | | | 3,0 | | |
| Parfuem | q.s. | q.s. | q.s. | q.s. | q.s. | q.s. | q.s. | q.s. |
| Natron- und/oder Kalilauge | q.s. | q.s. | q.s. | q.s. | q.s. | q.s. | q.s. | q.s. |
| Aqua Ad 100 | q.s. | Ad 100 | Ad 100 | Ad 100 | Ad 100 | Ad 100 | Ad 100 | Ad 100 |

Alle aufgeführten Beispielrezepturen ergeben eine außerordentlich stabile Emulsion mit Perlglanzoptik. Diese zeichnet sich im Gegensatz zu den Perlglanz-Emulsionen des Standes der Technik durch sehr gute Hautpflegeeigenschaften, gute Hautverträglichkeit und sensorisch ausgewogene, kosmetische Eigenschaften aus.

## Patentansprüche

1. Kosmetische oder dermatologische Zubereitung umfassend
(I) bis zu 12 Gew.%, bezogen auf das Gesamtgewicht der Zubereitungen, einer oder mehrerer C₁₂-C₄₀ Fettsäuren,
(II) 0 bis maximal 3 Gew.%, bezogen auf das Gesamtgewicht der Zubereitungen, einer oder mehrerer C₁₂-C₄₀ Fettalkohole
(III) 0,01 bis 10 Gew.%, bezogen auf das Gesamtgewicht der Zubereitungen, amphiphile Polymere und/oder assoziative Polymere und/oder Siloxane Elastomere und
(IV) Natronlauge und/oder Kalilauge

2. Kosmetische oder dermatologische Zubereitung umfassend
(I) maximal 10 Gew.%, bezogen auf das Gesamtgewicht der Zubereitungen, einer oder mehrerer C₁₂-C₄₀ Fettsäuren,
(II) 0,1 bis 10 Gew.%, bezogen auf das Gesamtgewicht der Zubereitungen, einer oder mehrerer C₁₂-C₄₀ Fettalkohole,
(III) 0,01 bis 10 Gew.%, bezogen auf das Gesamtgewicht der Zubereitungen, amphiphile Polymere und/oder assoziative Polymere und/oder Siloxane Elastomere,
(IV) Natronlauge und/oder Kalilauge sowie
(V) 0,1 - 10 Gew.%, bezogen auf das Gesamtgewicht der Zubereitungen, C₁₂-C₄₀ polyethoxylierte (POE) Fettsäureester mit einer POE-Kettenlänge von 10-100.

3. Zubereitungen nach Anspruch 2, **dadurch gekennzeichnet, dass** zusätzlich (VI) niedermolekulare Tenside enthalten sind.

4. Zubereitung nach Anspruch 1, 2 oder 3 , **dadurch gekennzeichnet, dass** folgende Verbindungen gewählt werden
(I) Stearinsäure und/oder Palmitinsäure,
(II) Myristyl Alkohol, Cetyl Alkohol, Behenyl Alkohol, Stearyl Alkohol und/oder Cetearyl Alkohol,
(III) Dimethicon/Vinyl Dimethicon Crosspolymer, Polysilicone -11, Acrylat/C10-30 Alkyl Acrylat Crosspolymer, Acrylat/Vinyl Isodecanoat Crosspolymer, Acrylat/Steareth - 20 Methacrylat Copolymer, Acrylat/Steareth -20 Itaconat Copolymer, Acrylat/Steareth-50 Acrylat Copolymer, Acrylat /Palmeth- 25 Acrylat Copolymer, Steareth-10 Ally Ether/Acrylat Copolymer, PEG-120 Methylglucose Dioleat, PEG-60 Sorbitan Tetraoleat, PEG-150 Pentaerythrityl Tetrasteart, PEG-55 Propylen Glycol Oleat, PEG-150 Distearat und/oder PEG-180 / Laureth-50 / TMMG Copolymer
(IV) Natronlauge
(V) gegebenenfalls PEG-30-Stearat, PEG-40-Stearat und/oder PEG-1 00 Stearat
(VI) gegebenenfalls Steareth-2, Laureth-4 und/oder Ceteth-3

5. Zubereitung nach Anspruch 2 oder 3, **dadurch gekennzeichnet, dass** das Verhältnis der Bestandteile I: II: V 5:1:1 bis 1:1:5, bevorzugt 3 : 1 : 1 bis 3 : 1 : 3, insbesondere bevorzugt 3 : 1 : 1 bis 1 : 1 : 3 beträgt.

6. Zubereitung nach einem der vorstehenden Ansprüche, **dadurch gekennzeichnet, dass** Natronlauge als ausschließliches Neutralisationsmittel enthalten ist.

7. Zubereitung nach einem der vorstehenden Ansprüche, **dadurch gekennzeichnet, dass** als Fettsäuren Stearinsäure und/oder Palmitinsäure enthalten sind.

8. Zubereitung nach einem der vorstehenden Ansprüche, **dadurch gekennzeichnet, dass** der verseifte Anteil der Fettsäuren max. 9% beträgt.

9. Zubereitung nach einem der Ansprüche 3 bis 8, **dadurch gekennzeichnet, dass** als niedermolekulares Tensid Laureth-4 enthalten ist.

10. Zubereitung nach einem der vorstehenden Ansprüche, **dadurch gekennzeichnet, dass** unpolare Lipide mit einer Polarität von mindestens 30 mN/m, Mineralöle, Silikonöle und/oder Wachse bis zu 30 Gew.%, bezogen auf die Gesamtmasse der Zubereitung, enthalten sind.

11. Zubereitung nach einem der vorstehenden Ansprüche, **dadurch gekennzeichnet, dass** die unpolaren Lipide mit einer Polarität von mindestens 30 mN/m oder Wachse gewählt werden aus der Gruppe der unpolaren Kohlenwasserstoffen, hydriertem Polyisobuten, Squalan, Silikonöle wie Cyclomethicone, Dimethicone, Wachse wie Methylpalmitat und/oder Dimethiconol Stearat.

12. Zubereitung nach den genannten Ansprüchen, **dadurch gekennzeichnet, dass** die Lipidphase bis zu 60 Gew.-% - bezogen auf das Gesamtgewicht der Lipidphase - polare Lipide mit einer Polarität von höchstens 30 mN/m enthält.

13. Zubereitungen nach einem der vorstehenden Ansprüche, **dadurch gekennzeichnet, dass** zusätzlich Lösungsvermittler enthalten sind, insbesondere PEG-40 Hydrogenated Castor Oil.

14. Zubereitung nach einem der vorstehenden Ansprüche, **dadurch gekennzeichnet, dass** zusätzlich Lichtschutzfilter, Moisturizer, Wirkstoffe, Puderrohstoffe, Konservierungsmittel, Farbstoffe, Füllstoffe und/oder Deodorantien enthalten sind.

15. Zubereitung nach den genannten Ansprüchen, **dadurch gekennzeichnet, daß** Ethanol bis 30 Gew.% - bezogen auf das Gesamtgewicht der Zubereitung - enthalten ist.

16. Verwendung der kosmetischen oder dermatologischen Zubereitung nach einem der vorstehenden Ansprüche zur Erzielung eines Perlglanzeffektes.

17. Verwendung der kosmetischen oder dermatologischen Zubereitung nach Anspruch 16 in dekorativen Kosmetika.

18. Verwendung nach Anspruch 16 oder 17 in Hautpflegezubereitungen, Cremes, Lotions, Fluids, Make-up, Sprays, Mousses, Aerosolen und/oder Lichtschutzmitteln sowie Reinigungsemulsionen oder Masken.
